# EUROPÄISCHE PATENTSCHRIFT

(11) **EP 0 367 205 B1**
(45) Veröffentlichungstag und Bekanntmachung des Hinweises auf die Patenterteilung: **29.12.1993**
(21) Anmeldenummer: 89120142.8
(22) Anmeldetag: 30.10.1989
(51) Int. Cl.: C07C 229/22, C07C 229/12, C07C 229/36, C07C 229/24, A61K 31/195

(54) **2-Aminocarbonsäuren und deren Derivate, Verfahren zu deren Herstellung und deren Verwendung als Arzneimittel**
2-Aminocarboxylic acids and their derivatives, methods for their preparation and their use as pharmaceutical compositions
Acides 2-aminocarboxyliques et leurs dérivés, procédés pour leur préparation et leur utilisation comme compositions pharmaceutiques

(30) Priorität: 31.10.1988 DE 3836987
(43) Veröffentlichungstag der Anmeldung: 09.05.1990
(73) Patentinhaber: GÖDECKE AKTIENGESELLSCHAFT, D-10587 Berlin (DE)
(72) Erfinder: Trostmann, Dr. Uwe, D-7806 March-Hugstetten (DE); Hartenstein, Dr. Johannes, D-7801 Stegen-Wittental (DE); Rudolph, Dr. Claus, D-7801 Vörstetten (DE); Schächtele, Dr. Christoph, D-7800 Freiburg (DE); Osswald, Prof. Dr. Hartmut, D-7808 Waldkirch 2 (DE); Weinheimer, Dr. Günter, D-7819 Denzlingen (DE)

(56) Entgegenhaltungen:
- EP-A- 0 053 434
- DE-A- 1 518 362
- DE-A- 1 617 077
- DE-A- 2 025 573
- DE-A- 2 940 509
- GB-A- 727 482
- US-A- 3 647 868
- US-A- 4 358 368
- CHEMICAL ABSTRACTS, Band 66, Nr. 15, 10. April 1967, Seite 6335, Zusammenfassung Nr. 67040u, Columbus, Ohio, US; E. ULSPERGER: "Anionic surfactants from amino acids and glycidyl ethers"
- CHEMICAL ABSTRACTS, Band 104, Nr. 24, 16. Juni 1986, Seite 201, Zusammenfassung Nr. 210577k, Columbus, Ohio, US; M. HELLSTEN et al.: "Froth-flotation collectors", & BR-A-85 01 477

## Beschreibung

Die Erfindung betrifft eine neue Indikation von 2-Aminocarbonsäuren und Derivaten der allgemeinen Formel Ia, 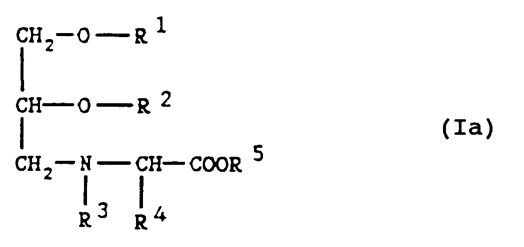
in welcher R¹ eine geradkettige oder verzweigte, gesättigte oder ungesättigte Alkylgruppe mit bis zu 20 Kohlenstoffatomen, R² und R³, die gleich oder verschieden sein können, Wasserstoff oder eine geradkettige oder verzweigte Alkylgruppe mit bis zu 4 Kohlenstoffatomen, R⁴ Wasserstoff, Methyl, Benzyl, Hydroxybenzyl, eine Carboxylgruppe, eine Hydroxyalkyl- oder Alkoxycarbonylgruppe mit jeweils bis zu 4 C-Atomen und R⁵ Wasserstoff oder eine geradkettige oder verzweigte Alkylgruppe mit bis zu 4 Kohlenstoffatomen bedeutet, sowie deren pharmakologisch unbedenkliche Salze.

Die Erfindung betrifft weiterhin neue Verbindungen der allgemeinen Formel Ib 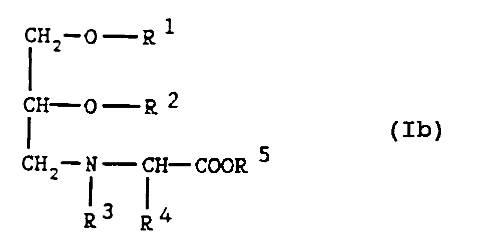
in welcher R¹ eine geradkettige oder verzweigte, gesättigte oder ungesättigte Alkylgruppe mit bis zu 20 Kohlenstoffatomen, R² und R³ die gleich oder verschieden sein können und Wasserstoff oder eine geradkettige oder verzweigte Alkylgruppe mit bis zu 4 Kohlenstoffatomen, R⁴ Benzyl, Hydroxybenzyl, eine Carboxylgruppe, eine Hydroxyalkyl- oder Alkoxycarbonylgruppe mit jeweils bis zu 4 C-Atomen und R⁵ Wasserstoff oder eine geradkettige oder verzweigte Alkylgruppe mit bis zu 4 Kohlenstoffatomen bedeutet, sowie deren pharmakologisch unbedenklichen Salze.

Bevorzugt werden 2-Aminocarbonsäuren und Derivate der allgemeinen Struktur Ib, in welcher R¹ eine Octyl- oder Octadecylgruppe, R² und R³ Wasserstoff oder eine Methylgruppe, R⁴ eine Hydroxymethyl-, Hydroxyethyl-, Benzyl-, Hydroxybenzyl- oder Ethoxycarbonylgruppe und R⁵ Wasserstoff oder eine niedere Alkylgruppe mit 1-4 C-Atomen darstellen.

Besonders bevorzugt wird die Verbindung 2-[N-Methyl-N-(3-octadecyloxy-2-hydroxypropyl)]amino-3-hydroxy-propionsäure.

Die Erfindung betrifft weiterhin ein Verfahren zur Herstellung von 2-Aminocarbonsäuren und Derivaten der allgemeinen Struktur Ib, das dadurch gekennzeichnet ist, daß entweder
a) Amino-Verbindungen der allgemeinen Formel II, 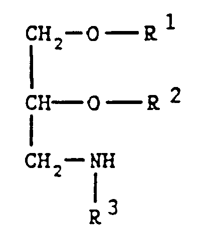 in der R¹, R² und R³ die oben angegebene Bedeutung besitzen, mit Verbindungen der allgemeinen Formel III, 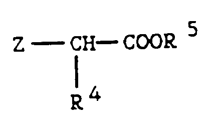 in der R⁴und R⁵ die angegebene Bedeutung haben, Z eine gute Abgangsgruppe, wie z.B. eine Tosylgruppe oder ein Halogenatom bedeutet, zu Verbindungen der allgemeinen Formel Ib umsetzt, oder
b) daß man Epoxide der allgemeinen Formel IV. 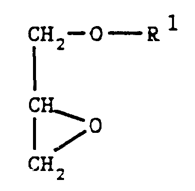 in der R¹ die oben angegebene Bedeutung hat, mit Verbindungen der allgemeinen Formel V, 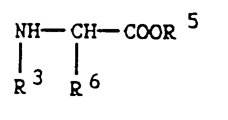 in der R³ und R⁵ die oben angegebene Bedeutung haben und R⁶ eine Benzyl- oder Carboxylgruppe oder eine chemische Gruppe der allgemeinen Formeln VI oder VIa,

   ―CH₂―O―R⁷ (VI)

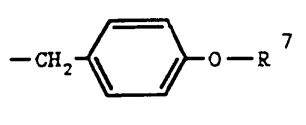

   in der R⁷ eine leicht abspaltbare Schutzgruppe, wie z.B. Benzyl, Chlorbenzyl, 2,6-Dichlorbenzyl oder tert. Butyl ist, zu Verbindungen der allgemeinen Formel VII, 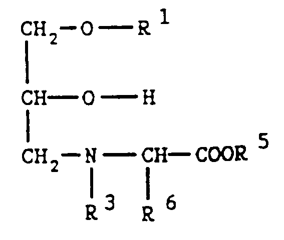 in der R¹, R³, R⁵ und R⁶ die oben angegebene Bedeutung besitzen, umsetzt, sodann, für den Fall, daß R² nicht Wasserstoff sein soll, die Verbindungen nach allgemein bekannten Verfahren zu Verbindungen der allgemeinen Formel VIII, 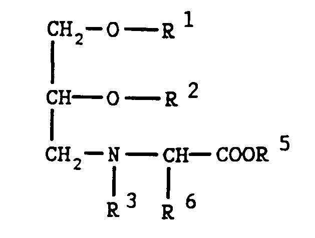 in der R¹, R², R³, R⁵ und R⁶ die oben angegebene Bedeutung besitzen, alkyliert und anschließend diese Verbindungen der allgemeinen Formel VIII durch hydrogenolytische oder saure Abspaltung der oben genannten Schutzgruppen in Verbindungen der allgemeinen Formel Ib überführt.

Die als Ausgangsverbindungen dienenden Aminoether der allgemeinen Formel II, 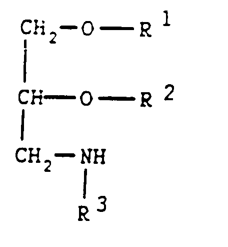
in der R¹, R² und R³ die oben angegebene Bedeutung besitzen und R² nicht Wasserstoff ist, werden hergestellt, indem man entweder
1) für den Fall, daß R² Methyl oder Ethyl bedeutet, die Salze der Aminoalkohole der allgemeinen Formel IX, 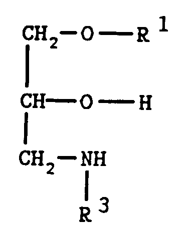 in der R¹ und R³ die oben angegebene Bedeutung besitzen, mit Trialkyloxoniumtetrafluorborat umsetzt, oder
2) Verbindungen der allgemeinen Formel X, 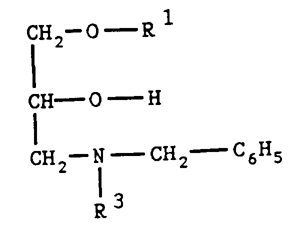 in der R¹und R³ die oben angegebene Bedeutung besitzen und R³ auch eine Benzylgruppe als Schutzgruppe sein kann, in die Natriumalkoholate überführt und diese mit Verbindungen der allgemeinen Formel XI,

   Y―R² (XI)

   in der R² die oben angegebene Bedeutung besitzt und Y eine gute Abgangsgruppe, wie z.B. einen Tosylrest oder ein Halogenatom, darstellt, zu Verbindungen der allgemeinen Formel XII, 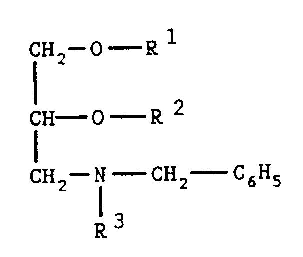 in der R¹, R² und R³ die oben angegebene Bedeutung besitzen, umsetzt und anschließend Verbindungen der allgemeinen Formel XII mit Wasserstoff in Gegenwart von Palladium in Verbindungen der allgemeinen Formel II, in der R¹, R² und R³ die oben angegebene Bedeutung besitzen, überführt.

Die Herstellung von Verbindungen der allgemeinen Formel IX ist in der Patentanmeldung EP 0255126 beschrieben und nicht Gegenstand dieser Anmeldung. Die für dieses Verfahren eingesetzten Derivate der Aminocarbonsäureester der allgemeinen Formel V sind entweder käuflich oder können nach allgemein bekannten Verfahren hergestellt werden (Houben-Weyl Bd. XI/2, S. 308 ff.).

Verbindungen der allgemeinen Formel Ia, bei denen R¹ eine geradkettige Alkylgruppe mit bis zu 18 Kohlenstoffatomen, R² Wasserstoff, R³ und R⁴ Wasserstoff oder einen niederen Alkylrest und R⁵ Wasserstoff bedeuten, sind bekannt und aufgrund ihrer Eigenschaften für dermatologische und kosmetische Präparate (DE-A-1518362) und als grenzflächenaktive Stoffe (DE-A-1518362, US-3647868, US-4358368, DD-60382; Fette, Seifen, Anstrichmittel 68, 964 (1966)) geeignet. Eine Wirkung auf die Protein Kinase C ist bislang noch nicht beschrieben.

Die Umsetzung der Aminoalkohole der allgemeinen Formel IX zu Aminoethern der allgemeinen Formel II kann durchgeführt werden entweder, indem man die Hydrochloride der Aminoalkohole IX mit einem Überschuß Trialkyloxoniumtetrafluorborat in aprotischen Lösungsmitteln wie z.B. Tetrahydrofuran oder Ether, vorzugsweise jedoch Dichlormethan, bei Temperaturen zwischen 0 und 25°C, vorzugsweise bei der Siedetemperatur des entsprechenden Lösungsmittels, umsetzt, oder indem man die Aminoalkohole der allgemeinen Formel X mit Natriumhydrid in aprotischen Lösungsmitteln wie Tetrahydrofuran oder Diethylether, vorzugsweise jedoch N,N-Dimethylformamid, bei Temperaturen zwischen 10 und 50°C, vorzugsweise bei Raumtemperatur, umsetzt und anschließend mit Verbindungen der allgemeinen Formel XI in demselben Lösungsmittel bei Temperaturen zwischen 20 und 50°C, vorzugsweise beim Siedepunkt des Lösungsmittel, zur Reaktion bringt. Die Reaktionsprodukte lassen sich mit bekannten Trennverfahren wie Kristallisation und/oder Chromatographie isolieren und reinigen.

Die gegebenenfalls erforderliche katalytische Hydrierung von N-Benzyl- bzw. N,N-Dibenzylverbindungen der allgemeinen Formel XII wird durchgeführt, indem man die entsprechenden Edukte in polaren Lösungsmitteln wie Methanol oder Ethanol löst und bevorzugt bei Atmosphären-Druck und Raumtemperatur in Gegenwart von Pd/C als Katalysator mit Wasserstoff sättigt. Die Gewinnung der Produkte der allgemeinen Formel II erfolgt durch Kristallisation der entsprechenden Salze, vorzugsweise der Hydrochloride.

Die Umsetzung der Verbindungen der allgemeinen Formel II mit Verbindungen der allgemeinen Formel III erfolgt, indem man die Ausgangsprodukte in einem polaren oder apolaren Lösungsmittel wie Isopropanol, N,N-Dimethylformamid oder Toluol, vorzugsweise jedoch Ethanol, bei Temperaturen zwischen 20 und 80°C, vorzugsweise beim Siedepunkt des Lösungsmittels, in Gegenwart von anorganischen oder organischen Basen wie zum Beispiel Natriumcarbonat, Piperidin oder Pyridin, vorzugsweise jedoch Kaliumcarbonat, reagieren läßt. Die Reaktionszeiten liegen im allgemeinen zwischen 1 und 24h, vorzugsweise jedoch bei 10h. Die Reindarstellung der Produkte der allgemeinen Formel Ib erfolgte durch Kristallisation und/oder Chromatographie.

Die Darstellung von Verbindungen der allgemeinen Formel VII erfolgt, indem man, nach bekannten Verfahren (CH-638 649; Fette, Seifen, Anstrichmittel 68, 964 (1966)) Epoxide der allgemeinen Formel IV mit Aminocarbonsäure-Derivaten der allgemeinen Formel V, in der R⁵ eine C₁₋₄-Alkylgruppe bedeutet, in einem organischen Lösungsmittel und/oder Wasser, bevorzugt jedoch Ethanol, bei Temperaturen zwischen 20 und 100°C, bevorzugt jedoch bei 50°C, umsetzt. Werden für diese Reaktion Aminocarbonsäuren der allgemeinen Struktur V (R⁵ = H) eingesetzt, so ist die Gegenwart einer anorganischen Base, bevorzugt Natriumhydroxid, erforderlich.

Verbindungen der allgemeinen Formel VII werden O-alkyliert, indem man diese Verbindungen in die Natriumalkoholate überführt und sodann in demselben Lösungsmittel, wie Tetrahydrofuran oder N,N-Dimethylformamid, mit Alkylverbindungen der allgemeinen Formel XI, bei Temperaturen zwischen 25 und 100°C, bevorzugt jedoch beim Siedepunkt des Lösungsmittels, umsetzt.

Die gegebenenfalls erforderliche Abspaltung einer Schutzgruppe R⁷ von Verbindungen der allgemeinen Formel VIII, in der R⁶ die Bedeutung einer chemischen Gruppe der allgemeinen Formel VI hat, wird entweder a) mittels katalytischer Hydrierung von O-Benzyl-Verbindungen der allgemeinen Formel VIII in polaren Lösungsmitteln wie Methanol, Ethanol, Essigsäure oder Wasser, bevorzugt jedoch in Gemischen aus Methanol/Essigsäure/Wasser 9:1:1, mit Wasserstoff in Gegenwart von Pd/C als Katalysator, durchgeführt, oder b) durch Reaktion der O-tert.-Butyl-Verbindungen der allgemeinen Struktur VIII mit starken Säuren wie Bromwasserstoffsäure oder Chlorwasserstoffsäure, bevorzugt jedoch mit Trifluoressigsäure, in Lösungsmitteln wie Ethanol, Wasser, Essigsäure, Dichlormethan oder auch in Reagenz als Lösungsmittel, bei Temperaturen zwischen 0 und 50°C, bevorzugt jedoch bei Raumtemperatur, durchgeführt. Die Gewinnung der Produkte erfolgt durch Chromatographie und/oder Kristallisation.

Da die erfindungsgemäßen Verbindungen der allgemeinen Formel I chirale Zentren aufweisen, liegen sie entweder als Diastereomerengemische, racemische Gemische oder in Form der Enantiomeren vor.

Die Verbindungen der Formel Ib werden zum Zwecke der Reinigung und aus galenischen Gründen bevorzugt in kristalline, pharmakologisch verträgliche Salze übergeführt. Die Salze werden in üblicher Weise durch Neutralisation der Basen mit entsprechenden anorganischen oder organischen Säuren erhalten. Als Säuren kommen z.B. Salzsäure, Schwefelsäure, Phosphorsäure, Bromwasserstoffsäure, Essigsäure, Weinsäure, Milchsäure, Zitronensäure, Äpfelsäure, Salicylsäure, Ascorbinsäure, Malonsäure, Fumarsäure, Oxalsäure oder Bernsteinsäure in Frage. Die Säureadditionssalze werden in der Regel in an sich bekannter Weise durch Mischen der freien Base oder deren Lösungen mit der entsprechenden Säure oder deren Lösungen in Wasser oder einem organischen Lösunsgsmittel, beispielsweise einem niederen Alkohol wie Methanol, Ethanol oder 2-Propanol oder einem niederen Keton wie Aceton oder 2-Butanon oder einem Ether wie Diethylether, Diisopropylether, Tetrahydrofuran oder Dioxan, erhalten.

Die erfindungsgemäßen Verbindungen der allgemeinen Formel Ia und Ib weisen interessante pharmakologische Eigenschaften auf. Sie stellen eine Substanzgruppe dar, welche die Protein Kinase C hemmt. Der Vorteil dieser Verbindungen gegenüber den in den Patentanmeldungen EP 0255126 (Beispiel Nr.6 der Tabelle 1) und US 4173641 (Beispiel Nr. 7) beschriebenen Verbindungen besteht darin, daß sie die Protein Kinase C mit höherer Potenz hemmen, während cyclo-AMP-abhängige Kinase, cyclo-GMP-abhängige Kinase und Myosin-light-chain Kinase weit weniger beeinflußt werden (siehe Tabelle 1).

Die Protein Kinase C spielt für die intrazelluläre Signaltransduktion eine wichtige Schlüsselrolle und ist eng verknüpft mit der Regulation von kontraktilen, sekretorischen und proliferativen Prozessen (Y. Nishizuka, Nature 308, 693-698 (1984)). Aufgrund dieser Eigenschaften können die erfindungsgemäßen Verbindungen zur Behandlung und Prävention von Herz- und Gefäßkrankheiten wie Thrombosen, Arteriosklerose, Hypertension, von Entzündungsprozessen, Allergien, Krebs, viraler Erkrankungen und bestimmten degenerativen Schäden des Zentralnervensystems verwendet werden.

Die erfindungsgemäßen Verbindungen der allgemeinen Formel Ia können in flüssiger oder fester Form oral oder parenteral appliziert werden. Als Injektionslösung kommt vor allem Wasser zur Anwendung, welches die bei Injektionslösungen üblichen Zusätze wie Stabilisierungsmittel, Lösungsvermittler oder Puffer enthält.

Derartige Zusätze sind z. B. Tartrat- und Citrat-Puffer, Ethanol, Komplexbildner (wie Äthylendiamin-tetraessigsäure und deren nicht-toxische Salze) sowie hochmolekulare Polymere (wie flüssiges Polyäthylenoxid) zur Viskositätsregulierung. Feste Trägerstoffe sind z. B. Stärke, Lactose, Mannit, Methylcellulose, Talkum, hochdisperse Kieselsäuren, hohermolekulare Fettsäuren (wie Stearinsäure), Gelatine, Agar-Agar, Calciumphosphat, Magnesiumstearat, tierische und pflanzliche Fette, feste hochmolekulare Polymere (wie Polyäthylenglykol); für orale Applikation geeignete Zubereitungen können gewünschtenfalls zusätzliche Geschmacks- und/oder Süßstoffe enthalten.

Die folgenden Beispiele dienen der näheren Erläuterung der Erfindung:

### Beispiel 1

### 2-[N-Methyl-N-(3-octadecyloxy-2-hydroxypropyl)]amino-3-hydroxy-propionsäure Hydrochlorid

Ein Reaktionsgemisch aus 2,0 g (5,1 mmol) (±)-3-Methylamino-1-octadecyloxy-2-propanol und 0,93 g (5,1 mmol) 2-Brom-3-hydroxy-propionsäureethylester wird zusammen mit 2,1 g (15,3 mmol) Kaliumcarbonat in 60 ml wasserfreiem Ethanol 4h unter Rückfluß erhitzt. Nach dieser Zeit werden noch einmal 0,9 g (5,1 mmol) Bromhydrin und 2,1 g (15,3 mmol) Kaliumcarbonat hinzugefügt und noch eine weitere Stunde am Rückfluß erwärmt. Man läßt anschließend auf Raumtemperatur abkühlen, filtriert und destilliert das Lösungsmittel im Vakuum ab. Den Rückstand nimmt man in 100 ml Dichlormethan auf und fügt bis zur vollständigen Lösung Methanol hinzu. Anschließend gibt man 30 ml 2N Salzsäure dazu und rührt kräftig 15 min lang durch, separiert sodann die organische Phase, trocknet sie über Natriumsulfat und destilliert davon das Lösungsmittel im Vakuum ab. Der Rückstand kristallisiert aus Diethylether. Schmp. 100-104°C
In analoger Weise werden erhalten:
2-[N-Methyl-N-(3-octyloxy-2-hydroxypropyl)]amino-3-hydroxy-propionsäure (1.a); Schmp: 177°C (Zers.)

### Beispiel 2

### 2-[N-Methyl-N-(3-octadecyloxy-2-hydroxypropyl)]amino-3-hydroxy-propionsäureethylester

Ein Reaktionsgemisch aus 300 mg (0,7 mmol) (±)-3-Methylamino-1-octadecyloxy-2-propanol und 180 mg (0,7 mmol) 2-Brom-3-hydroxy-propionsäureethylester wird zusammen mit 210 mg (1,4 mmol) Kaliumcarbonat in 10 ml wasserfreiem Ethanol 3h unter Rückfluß erhitzt. Die abgekühlte Reaktionslösung wird filtriert, im Vakuum vom Lösungsmittel befreit und in 50 ml Dichlormethan aufgenommen. Man filtriert vom Ungelösten und trennt das Produktgemisch säulenchromatographisch auf (Kieselgel, Dichlormethan/ Methanol 18:1). Mit der ersten Fraktion wird das Produkt isoliert. Schmp. 54-58°C (aus Pentan)

### Beispiel 3

### (±)-2-N-(3-Octadecyloxy-2-hydroxypropyl)amino-essigsäure Hydrochlorid

In eine Lösung von 660 mg (2 mmol) (±)-1-Octadecyloxy-2,3-epoxypropan in 10 ml Ethanol gibt man bei Raumtemperatur 610 mg (8.1 mmol) Glycin und fügt anschliessend 120 mg (3 mmol) Natriumhydroxid, gelöst in 10 ml Wasser, hinzu. Man erwärmt das Reaktionsgemisch 1h auf 80°C, läßt auf Raumtemperatur abkühlen, fügt 20 ml 2N Salzsäure und 100 ml Dichlormethan hinzu, rührt kräftig durch, separiert die organische Phase und trocknet sie über Natriumsulfat. Nach Abdestillieren des Lösungsmittels im Vakuum wird der Rückstand aus Ether umkristallisiert. Nach Absaugen und Trocknen der Kristalle im Vakuum wird das Produkt isoliert. Schmp. 65-72°C.

In analoger Weise werden erhalten:
2-N-(3-Octadecyloxy-2-hydroxypropyl)amino-3-phenyl-propionsäure (3.a); Schmp. 47-50°C;
2-N-(3-Octadecyloxy-2-hydroxypropyl)amino-3-(4-hydroxy)phenyl-propionsäure Hydrochlorid (3.b); Schmp. 182-183°C;
2-N-(3-Octadecyloxy-2-hydroxypropyl)aminomalonsäurediethylester Oxalat (3.c); Schmp: 130-132°C;

### Beispiel 4

### 2-N-(3-Octadecyloxy-2-hydroxypropyl)amino-3-hydroxy-propionsäure Hydrochlorid

640 mg (1,2 mmol) 2-N-(3-Octadecyloxy-2-hydroxypropyl)amino-3-tert. butyloxy-propionsäure Hydrochlorid werden in 15 ml Trifluoressigsäure 1h bei Raumtemperatur gerührt. Man destilliert sodann das Reagenz im Vakuum ab, nimmt den Rückstand in 50 ml Dichlormethan auf, fügt 20 ml einer gesättigten Lösung von Natriumhydrogencarbonat in Wasser hinzu und rührt 15 min bei Raumtemperatur. Den entstandenen Niederschlag saugt man ab, löst ihn in Methanol, fügt 20 ml 2N Salzsäure hinzu und destilliert nach 5 min Rühren das Lösungsmittel ab. Von der Mutterlauge wird die organische Phase separiert, mit 2N Salzsäure angesäuert, der entstandene Niederschlag durch Zugabe von Methanol gelöst, die organische Phase separiert, die wäßrige Phase noch einmal mit Dichlormethan extrahiert, die vereinigten organischen Phasen über Natriumsulfat getrocknet und das Produkt nach Abdestillieren des Lösungsmittels im Vakuum zusammen mit dem oben isolierten Niederschlag aus Ether ausgeführt. Das reine Produkt wird nach Absaugen isoliert Schmp. 62-67°C.

Das als Vorstufe eingesetzte 2-N-(3-Octadecyloxy-2-hydroxypropyl)amino-3-tert. butyloxy-propionsäure Hydrochlorid wird folgendermaßen hergestellt:
In eine Lösung von 125 mg (3,1 mmol) Natriumhydroxid in 20 ml Ethanol werden 0,5 g (3,1 mmol) L-O-tert. Butyl-serin gelöst und anschließend 1,0 g (3,1 mmol) (±)-1-Octadecyloxy-2,3-epoxypropan hinzugefügt. Nach Zugabe von 10 ml Wasser wird das Reaktionsgemisch 4h auf 80°C erwärmt. Anschließend werden 50 ml Wasser und 100 ml Dichlormethan hinzugefügt und das Gemisch wird mit 2N Salzsäure angesäuert. Man separiert die organische Phase, extrahiert die wäßrige Phase noch dreimal mit je 30 ml Dichlormethan, trocknet die vereinigten organischen Extrakte über Natriumsulfat und trennt nach Abdestillieren des Lösemittels im Vakuum den Rückstand säulenchromatographisch auf (Kieselgel, Dichlormethan/Methanol 9:1). Mit der vierten Fraktion wird das Produkt isoliert.

### Beispiel 5

### 2-N-(3-Octadecyloxy-2-methoxypropyl)amino-propionsäureethylester Oxalat

Ein Reaktionsgemisch von 390 mg (1 mmol) (±)-3-Amino-2-methoxy-1-octadecyloxy-propan, 180 mg (1 mmol) 2-Brom-propionsäureethylester und 300 mg (2,2 mmol) Kaliumcarbonat in 20 ml wasserfreiem Ethanol wird über 3h am Rückfluß erhitzt. Anschließend gibt man noch einmal 540 mg (2 mmol) 2-Brom-propionsäureethylester und 600 mg (4,4 mmol) Kaliumcarbonat hinzu und erwärmt weitere 4h zum Rückfluß. Die abgekühlte Lösung wird sodann filtriert, das Lösemittel davon im Vakuum abdestilliert und der Rückstand säulenchromatographisch aufgetrennt (Kieselgel, Dichlormethan/Methanol 36:1). Mit der ersten Fraktion wird das Produkt isoliert. Der Aminosäureester wird anschließend in 10 ml Ether mit 36 mg Oxalsäure versetzt, das Lösemittel davon im Vakuum abdestilliert und der Rückstand aus Ether/Cyclohexan zur Kristallisation gebracht. Schmp. 77-80°C.

Das als Vorstufe eingesetzte (±)-3-Amino-2-methoxy-1-octadecyloxypropan Hydrochlorid wird folgendermaßen hergestellt:
1,6 g (4 mmol) (±)-3-Amino-1-octadecyloxy-2-propanol Hydrochlorid werden zusammen mit 3,12 g (20,0 mmol) Trimethyloxoniumtetrafluorborat in 50 ml Dichlormethan über 24h zum Rückfluß erhitzt. Die abgekühlte Reaktionslösung wird mit 150 ml Dichlormethan verdünnt und vorsichtig mit 20 ml 2N Natronlauge versetzt. Man rührt 10 min kräftig durch, separiert die organische Phase, versetzt sie mit 30 ml 2N Salzsäure und rührt wiederum 15 min kräftig durch. Nach Abtrennen der organischen Phase, Trocknen über Natriumsulfat und Abdestillieren des Lösemittels im Vakuum wird das Produkt isoliert.

In analoger Weise wird erhalten:
2-N-(3-Octadecyloxy-2-methoxypropyl)amino-3-hydroxy-buttersäure (5.a); Schmp. 48-60°C.

### Beispiel 6

### 2-[N-Methyl-N-(2-methoxypropyl-3-octadecyloxy)]amino-3-hydroxy-propionsäure Hydrochlorid

Ein Gemisch aus 0,41 g (1 mmol) 1-Methylamino-2-methoxy-3-octadecyloxypropan Hydrochlorid, 0,20 g (1 mmol) 2-Brom-3-hydroxy-propionsäureethylester und 0,41 g (3 mmol) Kaliumcarbonat in 30 ml absolutem Ethanol wird 4 Stunden am Rückfluß gekocht. Dann erfolgt eine weitere Zugabe von 0,2 g (1 mmol) Bromhydrin und 0,27 g (2 mmol) Kaliumcarbonat. Es wird nochmals 6 Stunden gekocht und danach sofort heiß filtriert. Das Filtrat wird im Vakuum vom Lösungsmittel befreit, in Dichlormethan und wenig Methanol aufgenommen, 30 ml 2N Salzsäure zugesetzt und 15 min bei Raumtemperatur gerührt. Die organische Phase wird separiert und die wäßrige Phase wird 3 mal mit Dichlormethan extrahiert. Die vereinigten organischen Extrakte werden über Natriumsulfat getrocknet und das Lösungsmittel im Vakuum abdestilliert. Der Rückstand wird mit Ether und anschließend mit warmem Aceton behandelt. Schmp. 63-65°C.

Das als Vorstufe eingesetzte (±)-1-Methylamino-2-methoxy-3-octadecyloxypropan Hydrochlorid wird folgendermaßen hergestellt:
Ein Reaktionsgemisch aus 3,49 g (8,86 mmol) 1-Methylamino-3-octadecyloxypropan-2-ol und 6,5 g (44,6 mmol) Trimethyloxonium-tetrafluorborat, in 60 ml Dichlormethan, wird 24 Stunden unter Rückfluß erhitzt. Man verdünnt anschließend mit 100 ml Dichlormethan, fügt vorsichtig 20 ml 2N Natronlauge hinzu, separiert die organische Phase, fügt hierzu 50 ml 2N Salzsäure, rührt 10 min. kräftig durch und separiert die organische Phase. Nach dem Trocknen und Abdestillieren des Lösungsmittels im Vakuum wird der Rückstand aus n-Pentan ausgerührt.

Die folgenden Vergleichsversuche veranschaulichen die pharmakologische Wirksamkeit der erfindungsgemäßen Verbindungen der allgemeinen Formel I:
1. C-Kinase
   Das Enzym wird aus Rattenhirn oder glatten Muskel (Hühnermagen) gereinigt. Seine Aktivität wird bestimmt über den Einbau von Phosphor-32-markiertem Phosphat in Histon. Der Reaktionsansatz von 200 l enthält folgende Komponenten: 50 mM HEPES-NaOH, pH 7,5, 5 mM MgCl₂, 1 mM DTT, 4 M freies Ca²⁺, 10 M ATP, 1 g Phosphatidylserin, 0,2 g 1,2-Diolein sowie 40 g Histon H-1. Der Ansatz wird 4 min bei 30°C vorinkubiert und die Reaktion dann durch Zusatz von 5 nM PKC gestartet. Nach 5 min Inkubation bei 30°C wird die Reaktion mit 10% TCA gestoppt und die Proben dann abfiltriert. Der Phosphateinbau wird mittels Cerenkov-Zählung im Scintillationszähler bestimmt.
2. A-Kinase
   Die Messung der Aktivität erfolgt mit der kommerziell ver fügbaren katalytischen Untereinheit des Enzyms. Dabei wird der Einbau von Phosphor-32-markiertem Phosphat in Histon gemessen. Der Reaktionsansatz von 200 l enthält folgende Komponenten: 50 mM PIPES-NaOH, pH 7,5, 10 mM MgCl₂, 1 mM DTT, 40 M ATP sowie 50 g Histon-H-2B. Die Durchführung des Testes erfolgt wie bei der C-Kinase.
3. G-Kinase
   Das Enzym wird aus Rinderlunge gereinigt und seine Aktivität über den Einbau von Phosphor-32-markiertem Phosphat in Histon bestimmt. Im Testansatz von 200 l sind folgende Komponenten enthalten: 20 mM Tris-HCl, pH 7,4, 5 mM MgCl₂, 1 mM DTT, 10 M ATP, 10 M cGMP, 40 g BSA, 2% Glycerin sowie 10 g Histon II-A. Das Starten der Reaktion erfolgt durch Zusatz von 2,5 nM G-Kinase. Ansonsten erfolgt die Durchführung des Testes wie bei der C-Kinase.
4. MLC-Kinase
   Sowohl das Enzym als auch das Substrat (MLC) werden aus Hühnermagen gereinigt. Die Aktivitätsmessung erfolgt ebenfalls über den Einbau von Phosphor-32-markiertem Phosphat. Der Reaktionsansatz von 200 l enthält folgende Komponenten: 50 mM MOPS-NaOH, pH 7,2, 5 mM MgCl₂, 100 M CaCl₂, 100 nM Calmodulin, 1 mM DTT, 250 M ATP sowie 25 M MLC. Die Reaktion wird gestartet durch Zusatz von ca. 1 nM MLC-Kinase; ansonsten erfolgt die Durchführung des Testes wie bei der C-Kinase.

Die Ergebnisse der Vergleichsversuche sind in Tabelle 1 dargestellt.

**TABELLE I**

| Beispiel | Inhibierung von | | [IC₅₀-Werte (mol/l)] | |
|---|---|---|---|---|
| | PKC | cAMP-Kinase | cGMP-Kinase | MLC-Kinase |
| 1 | 2.5 x 10⁻⁶ | > 1 x 10⁻⁴ | > 1 x 10⁻⁴ | > 1 x 10⁻⁴ |
| A | 5.0 x 10⁻⁶ | > 1 x 10⁻⁴ | > 1 x 10⁻⁴ | > 1 x 10⁻⁴ |
| B | 7.0 x 10⁻⁶ | 2.9 x 10⁻⁵ | 2.8 x 10⁻⁵ | 5.1 x 10⁻⁶ |
| A: (±)-1-Dimethylamino-3-octadecyloxy-2-propylacetat Hydrochlorid (EP 0255126) B: 4-Aminomethyl-1-[2,3-(di-n-decyloxy)-n-propyl]-4-phenylpiperidin (US 4173641) PKC: Protein Kinase C cAMP-Kinase: cyclo-AMP-abhängige Kinase cGMP-Kinase: cyclo-GMP-abhängige Kinase MLC -Kinase: Myosin-light-chain Kinase | | | | |

## Patentansprüche

1. 2-Aminocarbonsäuren und deren Derivate der allgemeinen Formel Ib 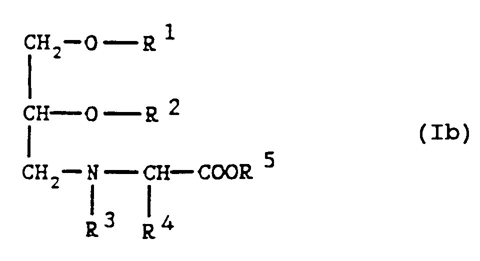 in welcher R¹ eine geradkettige oder verzweigte, gesättigte oder ungesättigte Alkylgruppe mit bis zu 20 Kohlenstoffatomen, R² und R³ die gleich oder verschieden sein können und Wasserstoff oder eine geradkettige oder verzweigte Alkylgruppe mit bis zu 4 Kohlenstoffatomen, R⁴ Benzyl-, Hydroxybenzyl, eine Carboxylgruppe, eine Hydroxyalkyl- oder Alkoxycarbonylgruppe mit jeweils bis zu 4 C-Atomen und R⁵ Wasserstoff oder eine geradkettige oder verzweigte Alkylgruppe mit bis zu 4 Kohlenstoffatomen bedeutet, sowie deren pharmakologisch unbedenkliche Salze.

2. Verbindungen der allgemeinen Formel Ib gemäß Anspruch 1, in welchen R¹ eine Octyl- oder Octadecylgruppe, R² und R³, die gleich oder verschieden sein können und Wasserstoff oder eine Methylgruppe, R⁴ eine Benzyl-, Hydroxymethyl-, Hydroxyethyl-, Hydroxybenzyl- oder Ethoxycarbonylgruppe und R⁵ Wasserstoff oder eine Ethylgruppe bedeutet.

3. Verbindungen der allgemeinen Formel Ib gemäß Anspruch 1, nämlich 2-[N-Methyl-N-(3-octadecyloxy-2-hydroxypropyl)]amino-3-hydroxy-propionsäure · Hydrochlorid; 2-[N-Methyl-N-(3-octyloxy-2-hydroxypropyl)]amino-3-hydroxy-propionsäure; 2-[N-Methyl-N-(3-octadecyloxy-2-hydroxypropyl)]amino-3-hydroxy-propionsäureethylester; 2-N-(3-Octadecyloxy-2-hydroxypropyl)amino-3-hydroxy-propionsäure · Hydrochlorid; 2-N-(3-Octadecyloxy-2-methoxypropyl)amino-3-hydroxybuttersäure; 2-N-(3-Octadecyloxy-2-hydroxypropyl)amino-3-(4-hydroxy)phenyl-propionsäure · Hydrochlorid; 2-N-(3-Octadecyloxy-2-hydroxypropyl)amino-malonsäurediethylester · Oxalat und 2-N-(3-Octadecyloxy-2-hydroxypropyl)amino-3-phenyl-propionsäure.

4. Verfahren zur Herstellung von 2-Aminocarbonsäuren und deren Derivate der allgemeinen Struktur Ib 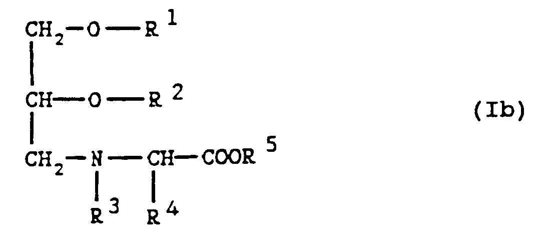 in welcher R¹ eine geradkettige oder verzweigte, gesättigte oder ungesättigte Alkylgruppe mit bis zu 20 Kohlenstoffatomen, R² und R³ die gleich oder verschieden sein können und Wasserstoff oder eine geradkettige oder verzweigte Alkylgruppe mit bis zu 4 Kohlenstoffatomen, R⁴ Benzyl-, Hydroxybenzyl, eine Carboxylgruppe, eine Hydroxyalkyl- oder Alkoxycarbonylgruppe mit jeweils bis zu 4 C-Atomen und R⁵ Wasserstoff oder eine geradkettige oder verzweigte Alkylgruppe mit bis zu 4 Kohlenstoffatomen bedeutet, sowie von deren pharmakologisch unbedenklichen Salzen, dadurch gekennzeichnet, daß man entweder
a) Amino-Verbindungen der allgemeinen Formel II, 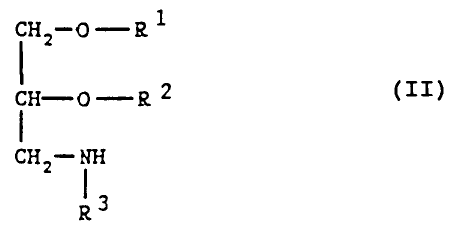 in der R¹, R² und R³ die oben angegebene Bedeutung besitzen, mit Verbindungen der allgemeinen Formel III, 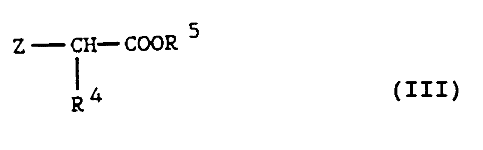 in der R⁴ und R⁵ die angegebene Bedeutung haben, Z eine gute Abgangsgruppe, wie z. B. eine Tosylgruppe oder ein Halogenatom bedeutet, zu Verbindungen der allgemeinen Formel Ib umsetzt, oder
b) daß man Epoxide der allgemeinen Formel IV, 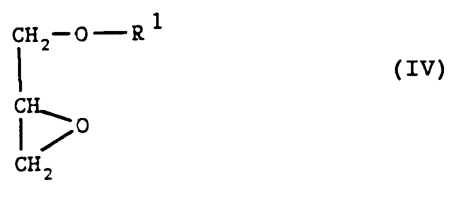 in der R¹ die oben angegebene Bedeutung hat, mit Verbindungen der allgemeinen Formel V, 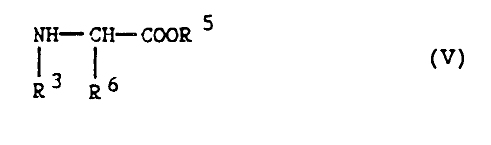 in der R³ und R⁵ die oben angegebene Bedeutung haben und R⁶ eine Benzyl- oder Carboxylgruppe oder eine chemische Gruppe der allgemeinen Formeln VI oder VIa,
―CH₂―O―R⁷ (VI) 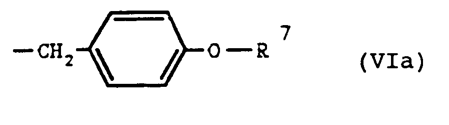
in der R⁷ eine leicht abspaltbare Schutzgruppe, wie z. B. Benzyl, Chlorbenzyl, 2,6-Dichlorbenzyl oder tert. Butyl ist, zu Verbindungen der allgemeinen Formel VII, 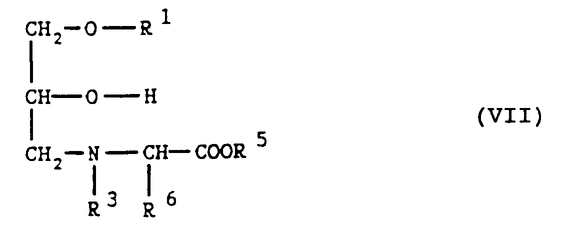 in der R ¹, R³, R⁵ und R⁶ die oben angegebene Bedeutung besitzen, umsetzt, sodann, für den Fall, daß R² nicht Wasserstoff sein soll, die Verbindungen nach allgemein bekannten Verfahren zu Verbindungen der allgemeinen Formel VIII 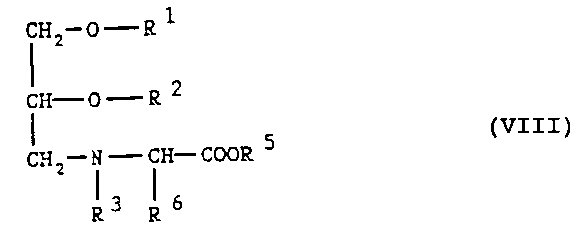 in der R¹, R², R³, R⁵ und R⁶ die oben angegebene Bedeutung besitzen, alkyliert und anschließend diese Verbindungen der allgemeinen Formel VIII durch hydrogenolytische oder saure Abspaltung der oben genannten Schutzgruppen in Verbindungen der allgemeinen Formel Ib überführt.

5. Verfahren nach Anspruch 4 zur Herstellung von Verbindungen der allgemeinen Formel I gemäß Anspruch 1, in welchen R¹ eine Octyl- oder Octadecylgruppe, R² und R³, die gleich oder verschieden sein können und Wasserstoff oder eine Methylgruppe, R⁴ eine Benzyl-, Hydroxymethyl-, Hydroxyethyl-, Hydroxybenzyl- oder Ethoxycarbonylgruppe und R⁵ Wasserstoff oder eine Ethylgruppe bedeutet.

6. Verfahren nach Anspruch 4 oder 5 zur Herstellung von Verbindungen der allgemeinen Formel I gemäß Anspruch 1, nämlich 2-[N-Methyl-N-(3-octadecyloxy-2-hydroxypropyl)]-amino-3-hydroxy-propionsäure · Hydrochlorid; 2-[N-Methyl-N-(3-octyloxy-2-hydroxypropyl)]amino-3-hydroxy-propionsäure; 2-[N-Methyl-N-(3-octadecyloxy-2-hydroxypropyl)]amino-3-hydroxy-propionsäureethylester; 2-N-(3-Octadecyloxy-2-hydroxypropyl)amino-3-hydroxy-propionsäure · Hydrochlorid; 2-N-(3-Octadecyloxy-2-methoxypropyl)amino-3-hydroxybuttersäure; 2-N-(3-Octadecyloxy-2-hydroxypropyl)amino-3-(4-hydroxy)phenyl-propionsäure · Hydrochlorid; 2-N-(3-Octadecyloxy-2-hydroxypropyl)amino-malonsäurediethyl-ester · Oxalat und 2-N-(3-Octadecyloxy-2-hydroxypropyl)amino-3-phenyl-propionsäure.

7. Verwendung von 2-Aminocarbonsäuren und Derivaten der allgemeinen Formel Ia, 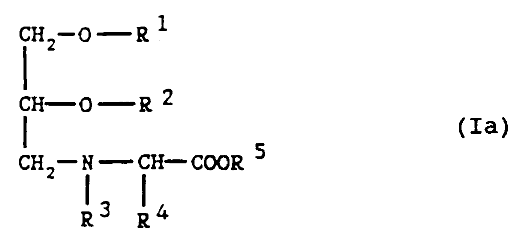 in welcher R¹ eine geradkettige oder verzweigte, gesättigte oder ungesättigte Alkylgruppe mit bis zu 20 C-Atomen, R² und R³ die gleich oder verschieden sein können und Wasserstoff oder eine geradkettige oder verzweigte Alkylgruppe mit bis zu 4 Kohlenstoffatomen, R⁴ Wasserstoff, Methyl, Benzyl, Hydroxybenzyl, eine Carboxylgruppe, eine Hydroxyalkyl- oder Alkoxycarbonylgruppe mit jeweils bis zu 4 C-Atomen und R⁵ Wasserstoff oder eine geradkettige oder verzweigte Alkylgruppe mit bis zu 4 Kohlenstoffatomen bedeutet, sowie deren pharmakologisch unbedenkliche Salze zur Herstellung von Arzneimitteln die zur Inhibierung der Proteinkinase C und damit zur Regulation von kontraktilen, sekretorischen und proliferativen Prozessen geeignet sind.

8. Verwendung von Verbindungen der allgemeinen Formel Ia gemäß Anspruch 7 zur Herstellung von Arzneimitteln, die zur Prävention und/oder Behandlung von Herz- und Gefäßkrankheiten wie Thrombosen, Arteriosklerose, Hypertension, von Entzündungsprozessen, Allergien, Krebs, viraler Erkrankungen und degenerativen Schäden des Zentralnervensystems geeignet sind.

9. Nicht dermatologische Arzneimittel enthaltend neben üblichen Hilfs- und Zusatzstoffen Verbindungen der allgemeinen Formel Ia gemäß Anspruch 7.

## Claims

1. 2-aminocarboxylic acids and their derivatives of the general formula Ib, 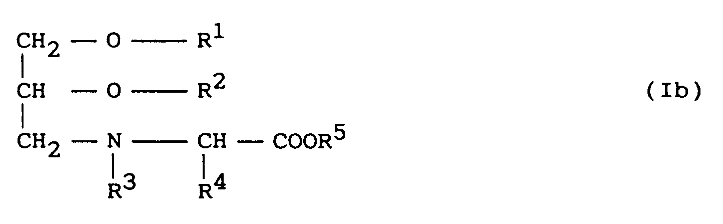 in which R¹ signifies a straight-chained or branched, saturated or unsaturated alkyl group with up to 20 carbon atoms, R² and R³ which can be the same or different signifies a hydrogen or a straight-chained or branched alkyl group with up to 4 carbon atoms, R⁴ benzyl-, hydroxybenzyl, a carboxyl group, a hydroxyalkyl group or alkoxycarbonyl group with in each case up to 4 C-atoms, and R⁵ hydrogen or a straight-chained or branched alkyl group with up to 4 carbon atoms, as well as their pharmacologically acceptable salts.

2. Compounds of the general formula Ib according to claim 1, in which R¹ signifies an octyl group or octadecyl group, R² and R³ which can be the same or different signifies a hydrogen or a methyl group, R⁴ a benzyl group, hydroxymethyl group, hydroxyethyl group, hydroxybenzyl group or ethoxycarbonyl group and R⁵ hydrogen or an ethyl group.

3. Compounds of the general formula Ib according to claim 1, namely, 2-[N-methyl-N-(3-octadecyloxy-2-hydroxypropyl)]-amino-3-hydroxy-propionic acid · hydrochloride; 2-[N-methyl-N-(3-octyloxy-2-hydroxypropyl)]amino-3-hydroxy-propionic acid; 2-[N-methyl-N-(3-octadecyloxy-2-hydroxypropyl)]amino-3-hydroxy-propionic acid ethyl ester; 2-N-(3-octadecyloxy-2-hydroxypropyl)amino-3-hydroxy-propionic acid · hydrochloride; 2-N-(3-octadecyloxy-2-methoxypropyl)amino-3-hydroxybutyric acid; 2-N-(3-octadecyloxy-2-hydroxypropyl)amino-3-(4-hydroxy)phenyl-propionic acid · hydrochloride; 2-N-(3-octadecyloxy-2-hydroxypropyl)amino-malonic acid diethyl ester · oxalate and 2-N-(3-octadecyloxy-2-hydroxypropyl)amino-3-phenyl-propionic acid.

4. Process for the production of 2-aminocarboxylic acids and their derivatives of the general structure Ib 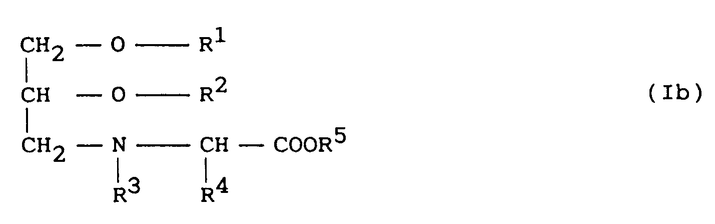 in which R¹ signifies a straight-chained or branched, saturated or unsaturated alkyl group with up to 20 carbon atoms, R² and R³ which can be the same or different is a hydrogen or a straight-chained or branched alkyl group with up to 4 carbon atoms, R⁴ benzyl-, hydroxybenzyl, a carboxyl group, a hydroxyalkyl group or alkoxycarbonyl group with in each case up to 4 C-atoms and R⁵ hydrogen or a straight-chained or branched alkyl group with up to 4 carbon atoms, as well as their pharmacologically acceptable salts, characterized in that either
a) amino compounds of the general formula II, 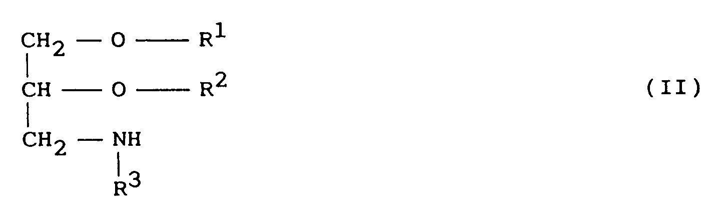 in which R¹, R² and R³ have the meaning indicated above, are caused to react with compounds of the general formula III, 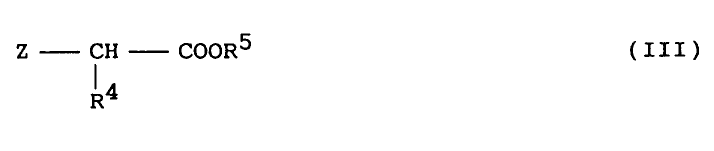 in which R⁴ and R⁵ have the indicated meaning, Z signifies a good removable group, for example a tosyl group or a halogen atom, to produce compounds of the general formula Ib, or
b) epoxides of the general formula IV, 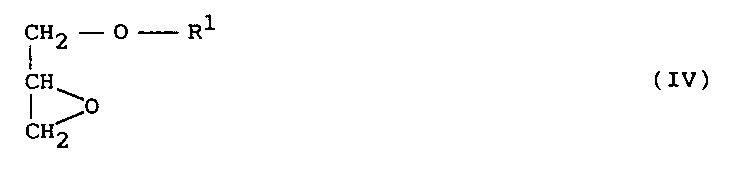 in which R¹ has the meaning indicated above, are caused to react with compounds of the general formula V, 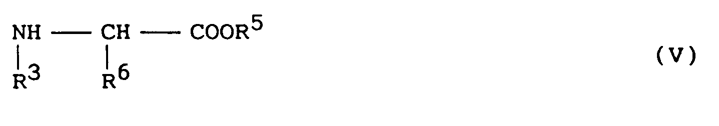 in which R³ and R⁵ have the meaning indicated above and R⁶ is a benzyl group or carboxyl group or a chemical group of the general formulae VI or VIa,
―CH₂―O―R⁷ (VI) 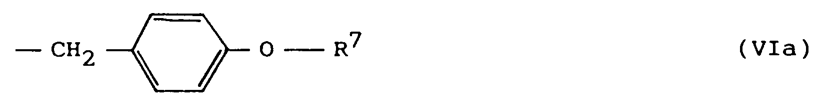
in which R⁷ is a protective group which can be easily split off, for example benzyl, chlorobenzyl, 2,6-dichlorobenzyl or tert. butyl, to produce compounds of the general formula VII 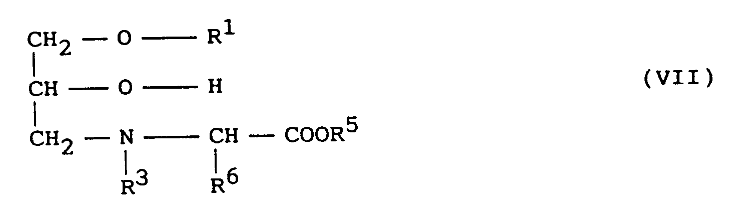 in which R¹, R³, R⁵ and R⁶ have the meaning indicated above, then, for the case in which R² is not to be hydrogen, the compounds are alkylated according to generally known methods into compounds of the general formula VIII, 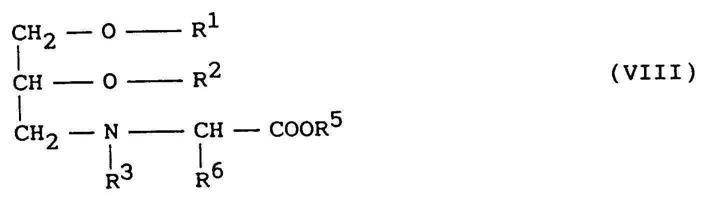 in which R¹, R², R³, R⁵ and R⁶ have the meaning indicated above, and subsequently these compounds of the general formula VIII are converted by hydrogenolytic or acid splitting off of the aforementioned protective groups into compounds of the general formula Ib.

5. Process according to claim 4 for the production of compounds of the general formula I according to claim 1, in which R¹ signifies an octyl group or octadecyl group, R² and R³ which can be the same or different is a hydrogen or a methyl group, R⁴ a benzyl group, hydroxymethyl group, hydroxyethyl group, hydroxybenzyl group or ethoxycarbonyl group and R⁵ hydrogen or an ethyl group.

6. Method according to claim 4 or 5 for the manufacture of compounds of the general formula I according to claim 1, namely 2-[N-methyl-N-(3-octadecyloxy-2-hydroxypropyl)]-amino-3 hydroxy-propionic acid · hydrochloride; 2-[N-methyl-N-(3-octyloxy-2-hydroxypropyl)]amino-3-hydroxy-propionic acid; 2-[N-methyl-N-(3-octadecyloxy-2-hydroxypropyl)]amino-3-hydroxy-propionic acid ethyl ester; 2-N-(3-octadecyloxy-2-hydroxypropyl)amino-3-hydroxy-propionic acid · hydrochloride; 2-N-(3-octadecyloxy-2-methoxypropyl)amino-3-hydroxybutyric acid; 2-N-(3-octadecyloxy-2-hydroxypropyl)amino-3-(4-hydroxy)phenyl-propionic acid · hydrochloride; 2-N-(3-octadecyloxy-2-hydroxypropyl)amino-malonic acid diethyl ester · oxalate and 2-N-(3-octadecyloxy-2-hydroxypropyl)amino-3-phenyl-propionic acid.

7. Use of 2-aminocarboxylic acids and derivatives of the general formula Ia, 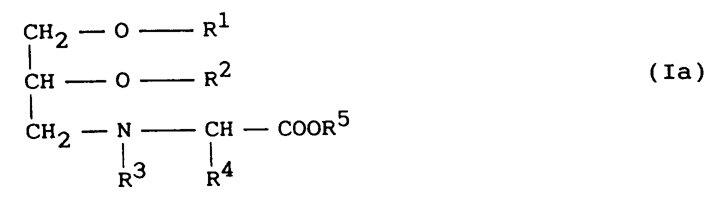 in which R¹ signifies a straight-chained or branched, saturated or unsaturated alkyl group with up to 20 C-atoms, R² and R³ which can be the same or different is a hydrogen or a straight-chained or branched alkyl group with up to 4 carbon atoms, R⁴ hydrogen, methyl, benzyl, hydroxybenzyl, a carboxyl group, a hydroxyalkyl group or alkoxycarbonyl group with in each case up to 4 C-atoms and R⁵ hydrogen or a straight-chained or branched alkyl group with up to 4 carbon atoms, as well as their pharmacologically acceptable salts for the manufacture of medicines which are suitable for the inhibition of protein kinase C and thus for the regulation of contractile, secretory and proliferative processes.

8. Use of compounds of the general formula Ia according to claim 7 for the manufacture of medicines which are suitable for the prevention and/or treatment of heart and vessel diseases such as thromboses, arteriosclerosis, hypertension, of inflammatory processes, allergies, cancer, viral illnesses and degenerative damage of the central nervous system.

9. Non-dermatological medicines containing, besides usual auxiliary and additive materials, compounds of the general formula Ia, according to claim 7.

## Revendications

1. Acides 2-aminocarboxyliques et leurs dérivés de formule générale Ib: 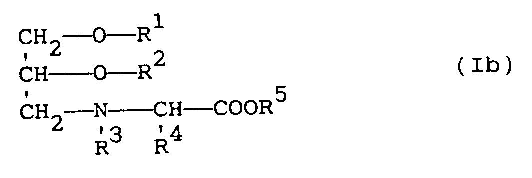 dans laquelle:
R¹ représente un groupe alkyle à chaîne droite ou ramifiée, saturé ou insaturé, renfermant jusqu'à 20 atomes de carbone,
R² et R³, qui peuvent être identiques ou différents, représentent un atome d'hydrogène ou un groupe alkyle à chaîne droite ou ramifiée renfermant jusqu'à 4 atomes de carbone,
R⁴ représente un radical benzyle, hydroxybenzyle, un groupe carboxyle, un groupe hydroxyalkyle ou un groupe alcoxycarbonyle renfermant chaque fois jusqu'à 4 atomes de carbone, et
R⁵ représente un atome d'hydrogène ou un groupe alkyle à chaîne droite ou ramifiée, renfermant jusqu'à 4 atomes de carbone,
ainsi que leurs sels pharmacologiquement acceptables.

2. Dérivés de formule générale Ib selon la revendication 1, dans lesquels:
R¹ représente un groupe octyle ou octadécyle,
R² et R³, qui peuvent être identiques ou différents, représentent un atome d'hydrogène ou un groupe méthyle,
R⁴ représente un groupe benzyle, hydroxyméthyle, hydroxyéthyle, hydroxybenzyle ou éthoxycarbonyle, et
R⁵ représente un atome d'hydrogène ou un groupe éthyle.

3. Dérivés de formule générale Ib selon la revendication 1, à savoir chlorhydrate d'acide 2-[N-méthyl-N-(octadécycloxy-2-hydroxypropyl)]-amino-3-hydroxy-propionique; acide 2-[N-méthyl-N-(3-octyloxy-2-hydroxypropyl)]-amino-3-hydroxy-propionique; ester éthylique d'acide 2-[N-méthyl-N-(3-octadécyloxy-2-hydroxypropyl)]-amino-3-hydroxy-propionique; chlorhydrate d'acide 2-N-(3-octadécyloxy-2-hydroxypropyl)amino-3-hydroxy-propionique; acide 2-N-(3-octadécyloxy-2-méthoxypropyl)-amino-3-hydroxybutyrique; chlorhydrate d'acide 2-N-(3-octadécyloxy-2-hydroxypropyl)amino-3-(4-hydroxy)phénylpropionique; oxalate d'ester diéthylique d'acide 2-N-(3-octadécyloxy-2-hydroxypropyl)amino-malonique et acide 2-N-(3-octadécyloxy-2-hydroxypropyl)amino-3-phénylpropionique.

4. Procédé de préparation d'acides 2-aminocarboxyliques et de dérivés de structure générale Ib: 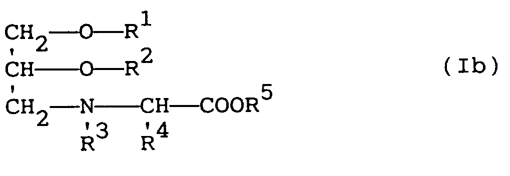 dans laquelle:
R¹ représente un groupe alkyle à chaîne droite ou ramifiée, saturé ou insaturé, renfermant jusqu'à 20 atomes de carbone,
R² et R³, qui peuvent être identiques ou différents, représentent un atome d'hydrogène ou un groupe alkyle à chaîne droite ou ramifiée renfermant jusqu'à 4 atomes de carbone,
R⁴ représente un groupe benzyle, hydroxybenzyle, un groupe carboxyle, un groupe hydroxyalkyle ou un groupe alcoxycarbonyle renfermant chaque fois jusqu'à 4 atomes de carbone, et
R⁵ représente un atome d'hydrogène ou un groupe alkyle à chaîne droite ou ramifiée, renfermant jusqu'à 4 atomes de carbone,
ainsi que de leurs sels pharmaceutiquement acceptables, caractérisé en ce que:
a) soit l'on fait réagir des dérivés aminés de formule générale II: 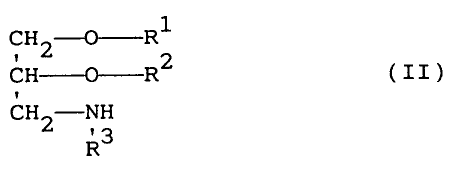 dans laquelle:
R¹, R² et R³ possèdent la signification indiquée ci-dessus,
avec les dérivés de formule générale III: 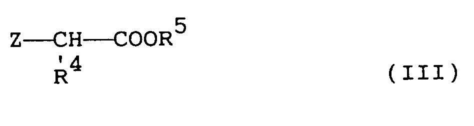 dans laquelle:
R⁴ et R⁵ ont la signification indiquée ci-dessus, et
Z représente un groupe de départ convenable comme par exemple un groupe tosyle ou un atome d'halogène,
pour obtenir des dérivés de formule générale Ib,
b) ou bien l'on fait réagir des époxydes de formule générale IV: 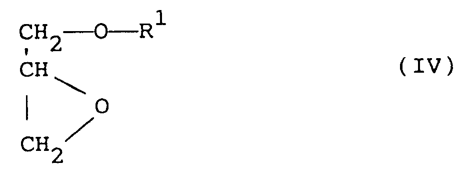 dans laquelle:
R¹ a la signification indiquée ci-dessus,
avec des dérivés de formule générale V: 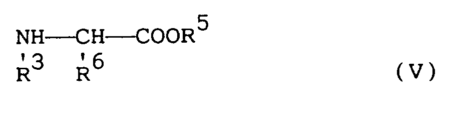 dans laquelle:
R³ et R⁵ ont la signification indiquée ci-dessus, et
R⁶ représente un groupe benzyle ou carboxyle ou un groupe chimique de formule générale VI ou VI:
―CH₂―O―R⁷ (VI) 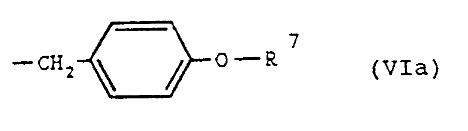
dans laquelle:
R⁷ représente un groupe protecteur facilement clivable tel que par exemple un radical benzyle, chlorobenzyle, 2,6-dichlorobenzyle ou tertiobutyle,
pour obtenir des dérivés de formule générale VII: 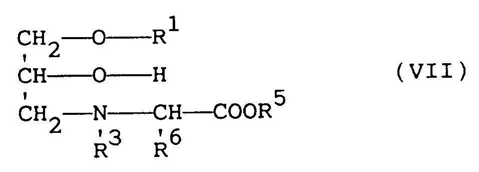 dans laquelle:
R¹, R³, R⁵ et R⁶ possèdent la signification indiquée ci-dessus,
et ensuite, pour le cas où R² ne doit pas être un atome d'hydrogène, on alkyle les dérivés par des procédés connus en dérivés de formule générale VIII: 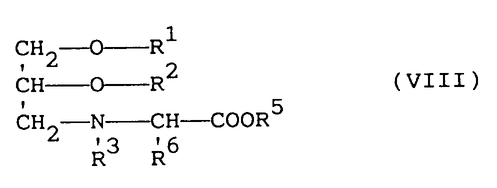 dans laquelle:
R¹, R², R³, R⁵ et R⁶ possèdent la signification indiquée ci-dessus,
et ensuite, on transforme ces dérivés de formule générale VIII par coupure hydrogénolytique ou acide des groupes protecteurs mentionnés ci-dessus en dérivés de formule générale Ib.

5. Procédé selon la revendication 4, de préparation de dérivés de formule générale I selon la revendication 1, dans lesquels:
R¹ représente un groupe octyle ou octadécyle,
R² et R³, qui peuvent être identiques ou différents, représentent un atome d'hydrogène ou un groupe méthyle,
R⁴ représente un groupe benzyle, hydroxyméthyle, hydroxyéthyle, hydroxybenzyle ou éthoxycarbonyle, et
R⁵ représente un atome d'hydrogène ou un groupe éthyle.

6. Procédé selon la revendication 4 ou 5, de préparation de dérivés de formule générale I selon la revendication 1, à savoir chlorhydrate d'acide 2-[N-méthyl-N-(3-octadécyloxy-2-hydroxypropyl)]-amino-3-hydroxy-propionique; acide 2-[N-méthyl-N-(3-octyloxy-2-hydroxypropyl)]-amino-3-hydroxy-propionique; ester éthylique d'acide 2-[N-méthyl-N-(3-octadécyloxy-2-hydroxypropyl)]-amino-3-hydroxy-propionique; chlorhydrate d'acide 2-N-(3-octadécyloxy-2-hydroxypropyl)amino-3-hydroxy-propionique; acide 2-N-(3-octadécyloxy-2-méthoxypropyl)-amino-3-hydroxybutyrique; chlorhydrate d'acide 2-N-(3-octadécyloxy-2-hydroxypropyl)amino-3-(4-hydroxy)phénylpropionique; oxalate d'ester diéthylique d'acide 2-N-(3-octadécyloxy-2-hydroxypropyl)amino-malonique et acide 2-N-(3-octadécyloxy-2-hydroxypropyl)amino-3-phénylpropionique.

7. Utilisation d'acides 2-aminocarboxyliques et de leurs dérivés de formule générale Ia: 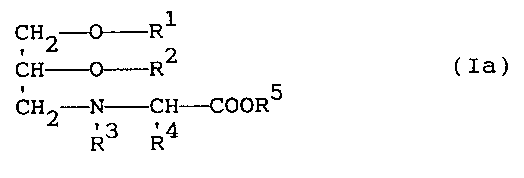 dans laquelle:
R¹ représente un groupe alkyle à chaîne droite ou ramifiée, saturé ou insaturé, renfermant jusqu'à 20 atomes de carbone,
R² et R³, qui peuvent être identiques ou différents, représentent un atome d'hydrogène ou un groupe alkyle à chaîne droite ou ramifiée renfermant jusqu'à 4 atomes de carbone,
R⁴ représente un atome d'hydrogène, un groupe méthyle, benzyle, hydroxybenzyle, un groupe carboxyle, un groupe hydroxyalkyle ou alcoxycarbonyle renfermant chaque fois jusqu'à 4 atomes de carbone, et
R⁵ représente un atome d'hydrogène ou un groupe alkyle à chaîne droite ou ramifiée, renfermant jusqu'à 4 atomes de carbone,
ainsi que de leurs sels pharmaceutiquement acceptables pour la préparation de médicaments qui conviennent à l'inhibition de la protéine kinase et de ce fait à la régulation de processus contractiles, sécrétoires et prolifératifs.

8. Utilisation des dérivés de formule générale Ia selon la revendication 7, pour la préparation de médicaments qui conviennent à la prévention et/ou au traitement de maladies cardiaques ou vasculaires comme thromboses, artériosclérose, hypertension, de processus inflammatoires, d'allergies, de cancers, de maladies virales et de dommages dégénératifs du système nerveux central.

9. Médicaments non dermatologiques contenant, outre des adjuvants et additifs usuels, des dérivés de formule générale Ia selon la revendication 7.
